# EUROPEAN PATENT APPLICATION

(11) **EP 3 699 858 A1**
(43) Date of publication of application: **26.08.2020**
(21) Application number: 19169106.2
(22) Date of filing: 12.04.2019
(51) Int. Cl.: G06Q 30/02, G06Q 30/06, G06Q 50/12

(54) **METHOD AND APPARATUS FOR FACILITATING DINING AT AN EATING ESTABLISHMENT**

(30) Priority: 19.02.2019 US 201962807350 P
(71) Applicant: Fuellgraf, Juergen, Orlando, FL 32819 (US)
(72) Inventor: Fuellgraf, Juergen, Orlando, FL 32819 (US)
(74) Representative: Stolmár & Partner Patentanwälte PartG mbB

(57) **Abstract**

A method is provided for facilitating dining at an eating establishment. The method includes the steps of capturing, with a camera, one or more images of an item on a menu at an eating establishment. The method also includes the step of performing the capturing step for each item on the menu at the eating establishment, for each menu at the eating establishment and for each eating establishment among a plurality of eating establishments. The method also includes the step of storing, in a database, the images captured with data indicating the item, the menu and the eating establishment for each stored image.

## Description

### CROSS REFERENCE TO RELATED APPLICATIONS

This application claims benefit of Provisional Application No. 62/807,350, filed February 19, 2019, under 35 U.S.C. § 119(e).

### BACKGROUND

Restaurants and other eating establishments routinely feature menus that provide a written description of each item or dish offered by the restaurant. However, it is difficult for restaurant patrons to visualize each item solely based on the written description of each item. In some restaurants, although an image of one or more items are sometimes placed in the menu, images are not included for each menu item.

Additionally, when a patron visits a restaurant in a foreign country the written description of the menu items is usually in a foreign language that is incomprehensible to the patron. Thus, in addition to an absence of images of each menu item, a restaurant patron in a foreign country also has no written description of the menu item to help understand the nature of the item.

### SUMMARY

It is here recognized that conventional methods used to facilitate display of images of menu items are deficient. For example, conventional methods are known where a patron at a restaurant uses a smartphone to scan data indicating the restaurant (e.g. a Quick Response Code or QR Code) which then links to social media applications (e.g. Facebook®, Twitter®) of users who shared photos of various items at the restaurant. However, the inventor of the present invention recognized that this conventional method is deficient since the linked photos, ingredients and/or allergy information do not reliably provide the patron with images, ingredients and/or allergy information of every menu item at the restaurant. Instead, the linked photos, ingredients and/or allergy information are confined to those menu items which various users of social media decided to order, capture photos or enter ingredient and/or allergy information about and share via the social media application. Those menu items that users ordered but failed to photograph or enter ingredient and/or allergy information about do not have images, ingredients and/or allergy information that are available for viewing in the social media application. Also, those menu item that users ordered and photographed but failed to share on the social media application do not have images that are available for viewing. Further, some users enter incorrect ingredient and/or allergy information for a menu item and thus such inaccurate information is then undesirably provided to a user using the social media application. Thus, the inventor of the present invention developed this method and apparatus to ensure that the restaurant patron is provided with an image of each and every menu item at the restaurant and/or is provided with accurate ingredient and/or allergy information of every menu item. In one example, the restaurant owner or staff provide the images, ingredients and/or allergy information of each menu item, to ensure accuracy of the information or images and the completeness of the images or information, e.g. that each menu item has available images and nutritional information. One advantage of the method is the reliability to the restaurant patron that each menu item has one or more images, ingredients and/or allergy information to be viewed.

In a first set of embodiments, a method is provided for facilitating dining at an eating establishment. The method includes the steps of capturing, with a camera, one or more images of an item on a menu at an eating establishment. The method also includes the step of performing the capturing step for each item on the menu at the eating establishment, for each menu at the eating establishment and for each eating establishment among a plurality of eating establishments. The method also includes the step of storing, in a database, the images captured with data indicating the item, the menu and the eating establishment for each stored image.

In another embodiment, the database includes a data structure having a plurality of records for a respective plurality of eating establishments in the network, where each record includes a plurality of fields for holding the data indicating the item, the menu and the eating establishment for each stored image.

In another embodiment, each record includes a first field for holding data indicating the eating establishment, a second field for holding data indicating the menu, a third field for holding data indicating the item and a fourth field holding the stored image data.

In another embodiment, the third field holds data indicating a description of the item in a plurality of languages.

In another embodiment, the third field holds data indicating at least one of a name of the item, a price of the item, a food category of the item, a rating of the item and nutritional information of the item.

In another embodiment, the food category includes one of vegan, vegetarian and non-vegetarian and where the nutritional information comprises ingredients, calories and information on fat, carbohydrates and protein.

In another embodiment, the first field is for holding a QR code indicating the eating establishment.

In another embodiment, the method further includes scanning, with a device, data indicating an eating establishment; transmitting, from the device to the database, the scanned data indicating the eating establishment; receiving, from the database to the device, the images of each item and each menu of the eating establishment based on the scanned data indicating the eating establishment; and displaying, on a screen of the device, one or more images of received images on the device.

In another embodiment, the scanning comprises scanning, with a camera of the device, a QR code of the eating establishment.

In another embodiment, the data indicating the item comprises data indicating a description of the item in a plurality of languages, where the method further includes: selecting, with the device, a language among a plurality of languages; transmitting, from the device to the database, the selected language; and receiving, from the database to the device, the description of each item and each menu of the eating establishment in the selected language.

In another embodiment, prior to step a, the method comprises: preparing, with a kitchen appliance at the eating establishment, a dish comprising the item so that the dish appears in a manner when served at the eating establishment.

In another embodiment, the method further includes transmitting, from a processor at each eating establishment, the images captured during steps a-c to the database over a network, where the processor and the database are connected over the network; and
where the storing the images in the database is performed after the images are transmitted to the database from the processor at each eating establishment.

In a second set of embodiments, a method is provided for displaying images of menu items from one of a plurality of eating establishments. The method includes scanning, with a device, data indicating one of the plurality of eating establishments. The method also includes transmitting, from the device to a database over a network, the scanned data. The method also includes receiving, from the database, one or more images of menu items at the eating establishment corresponding to the scanned data and data indicating the item, the menu and the eating establishment for each image. The method also includes displaying, in a first region of a screen of the device, the data indicating the eating establishment comprising a name of the eating establishment. The method also includes displaying, in a second region of the screen of the device, the data indicating the menu comprising a plurality of tabs indicating a respective plurality of menus of the eating establishment. The method also includes displaying in a third region of the screen of the device, one or more images of menu items corresponding to one of the menus in the second region.

In another embodiment, the method further includes selecting one of the tabs in the second region to select one of the menus and where the displaying in the third region comprises displaying one or more images of menu items corresponding to the selected tab.

In another embodiment, upon selecting one of the images in the third region, the method includes displaying, in the first region, the selected image of the menu item; displaying, in the second region, data indicating the item comprising at least one of a name of the item, a food category of the item and a rating of the item; displaying, in the third region, data indicating the item comprising a written description of the item in one of a plurality of languages; and
displaying, in a fourth region, data indicating the item comprising nutritional information comprising at least one of calories and ingredients.

In another embodiment, the method further comprises displaying a plurality of active areas corresponding to the plurality of languages and selecting one of the active areas corresponding to a selected language and where the displaying in the third region comprises displaying the written description in the selected language.

In a third set of embodiments, a system is provided for facilitating dining at an eating establishment. The system includes a camera at each of a plurality of eating establishments, where the camera is configured to capture one or more images of each item on each menu of the eating establishment. The system also includes a processor at each eating establishment that is communicatively coupled to the camera, and the processor is configured to receive the captured images from the camera. The system also includes a database communicatively coupled with the processor of each eating establishments to receive the one or more images and data indicating the item, the menu and the eating establishment from each processor. The database includes a plurality of records for the respective plurality of eating establishments, where each record includes a plurality of fields for holding the data indicating the item, the menu and the eating establishment for each stored image.

In another embodiment, the system further includes a device configured to scan data indicating the eating establishment and further configured to transmit the scanned data to the database; and a screen on the device configured to display one or more images received from the database of each item and each menu of the eating establishment based on the scanned data transmitted to the database.

In another embodiment, the device is a smartphone and where a camera of the smartphone is configured to scan a QR code indicating the eating establishment and where a screen of the smartphone is configured to display the one or more images.

In another embodiment, the system further includes a kitchen appliance at each of the
plurality of eating establishments , where the kitchen appliance is configured to prepare at least one item on at least one menu of the eating establishment.

Still other aspects, features, and advantages are readily apparent from the following detailed description, simply by illustrating a number of particular embodiments and implementations, including the best mode contemplated for carrying out the invention. Other embodiments are also capable of other and different features and advantages, and its several details can be modified in various obvious respects, all without departing from the spirit and scope of the invention. Accordingly, the drawings and description are to be regarded as illustrative in nature, and not as restrictive.

### BRIEF DESCRIPTION OF THE DRAWINGS

Embodiments are illustrated by way of example, and not by way of limitation, in the figures of the accompanying drawings in which like reference numerals refer to similar elements and in which:
FIG. 1 is a block diagram that illustrates an example system for facilitating dining at an eating establishment, according to an embodiment;
FIG. 2 is a block diagram that illustrates an example data structure used to store image data of the menu items of the eating establishment, according to an embodiment;
FIG. 3A is an image that illustrates an example of a login view on a display of a software application for facilitating dining at an eating experience, according to an embodiment;
FIG. 3B is an image that illustrates an example of a lost password view on the display of the software application of FIG. 3A, according to an embodiment;
FIG. 3C is an image that illustrates an example of a scan QR code view on the display of the software application of FIG. 3A, according to an embodiment;
FIG. 3D is an image that illustrates an example of a QR code scan area on the display of the software application of FIG. 3A, according to an embodiment;
FIG. 3E is an image that illustrates an example of a language selection view on the display of the software application of FIG. 3A, according to an embodiment;
FIG. 3F is an image that illustrates an example of an eating establishment view on the display of the software application of FIG. 3A, according to an embodiment;
FIG. 3G is an image that illustrates an example of an item view on the display of the software application of FIG. 3A, according to an embodiment;
FIG. 3H is an image that illustrates an example of a search view on the display of the software application of FIG. 3A, according to an embodiment;
FIG. 3I is an image that illustrates an example of a food category selection view on the display of the software application of FIG. 3A, according to an embodiment;
FIG. 3J is an image that illustrates an example of a review and rating view on the display of the software application of FIG. 3A, according to an embodiment;
FIG. 3K is an image that illustrates an example of a recent scan view on the display of the software application of FIG. 3A, according to an embodiment;
FIG. 3L is an image that illustrates an example of a rate product view on the display of the software application of FIG. 3A, according to an embodiment;
FIG. 4A is a flow diagram that illustrates an example of a method for capturing and storing image data of menu items of a plurality of eating establishments, according to an embodiment;
FIG. 4B is a flow diagram that illustrates an example of a method for displaying images of menu items from one of a plurality of eating establishments, according to an embodiment;
FIG. 5 is a block diagram that illustrates a computer system upon which an embodiment of the invention may be implemented;
FIG. 6 is a block diagram that illustrates a chip set upon which an embodiment of the invention may be implemented; and
FIG. 7 is a block diagram that illustrates a mobile terminal upon which an embodiment of the invention may be implemented;

### DETAILED DESCRIPTION

A method and apparatus are described for capturing and storing image data of each menu item at one or more eating establishments. A method and apparatus are also described for displaying images of menu items from one of a plurality of eating establishments. In the following description, for the purposes of explanation, numerous specific details are set forth in order to provide a thorough understanding of the present invention. It will be apparent, however, to one skilled in the art that the present invention may be practiced without these specific details. In other instances, well-known structures and devices are shown in block diagram form in order to avoid unnecessarily obscuring the present invention.

Notwithstanding that the numerical ranges and parameters setting forth the broad scope are approximations, the numerical values set forth in specific non-limiting examples are reported as precisely as possible. Any numerical value, however, inherently contains certain errors necessarily resulting from the standard deviation found in their respective testing measurements at the time of this writing. Furthermore, unless otherwise clear from the context, a numerical value presented herein has an implied precision given by the least significant digit. Thus, a value 1.1 implies a value from 1.05 to 1.15. The term "about" is used to indicate a broader range centered on the given value, and unless otherwise clear from the context implies a broader range around the least significant digit, such as "about 1.1" implies a range from 1.0 to 1.2. If the least significant digit is unclear, then the term "about" implies a factor of two, e.g., "about X" implies a value in the range from 0.5X to 2X, for example, about 100 implies a value in a range from 50 to 200. Moreover, all ranges disclosed herein are to be understood to encompass any and all sub-ranges subsumed therein. For example, a range of "less than 10" can include any and all sub-ranges between (and including) the minimum value of zero and the maximum value of 10, that is, any and all sub-ranges having a minimum value of equal to or greater than zero and a maximum value of equal to or less than 10, e.g., 1 to 4.

Some embodiments of the invention are described below in the context of dishes or items offered by eating establishments. In other embodiments, the invention is described in the context of dishes or items provided on a menu that is offered by eating establishments (e.g. restaurants). In other embodiments, the invention is described in the context of one or more retail stores that sell retail items, including a network of retail stores, e.g. car dealership, clothing store, grocery store, electronics store, hardware store, or any combination thereof, where the retail store includes indicia (e.g. QR code) that can be scanned by a device of a user (e.g. smart phone). In an embodiment, in response to scanning the indicia of the retail score, the embodiments of the present invention herein can be used to display one or more images and information about one or more items in stock at the retail store (e.g. a list of cars in stock at a dealership, a list of parameters of each car including the engine, the number of doors, etc., the price of each car).

As used herein the term "eating establishment" refers to any public place where food or drink are served for a fee, including but not limited to restaurants, coffee shops, cafes, diners, hotels, food trucks, bakeries, take outs, fast food chains, or bars including sports bars. It should be noted that "eating establishment" does not require that food is served at the establishment since it includes bars and coffee shops which may or may not exclusively sell beverages. As used herein, the term "item" means any food dish or drink selection offered by the eating establishment, either on a fixed menu or not on a menu (e.g. daily special). As used herein, the term "menu" means a group of items offered by the eating establishment that are grouped together on a list, e.g. breakfast menu, dinner menu, lunch menu, dessert menu. As used herein, "QR code" means a type of matrix barcode (or two-dimensional barcode) and where a "barcode" is a machine-readable optical label that contains information about the item to which it is attached. In practice, QR codes often contain data for a locator, identifier, or tracker that points to a website or application. A QR code uses four standardized encoding modes (numeric, alphanumeric, byte/binary, and kanji) to store data efficiently; extensions may also be used. A QR code consists of black squares arranged in a square grid on a white background, which can be read by an imaging device such as a camera, and processed using Reed-Solomon error correction until the image can be appropriately interpreted. The required data is then extracted from patterns that are present in both horizontal and vertical components of the image

### 1. Overview

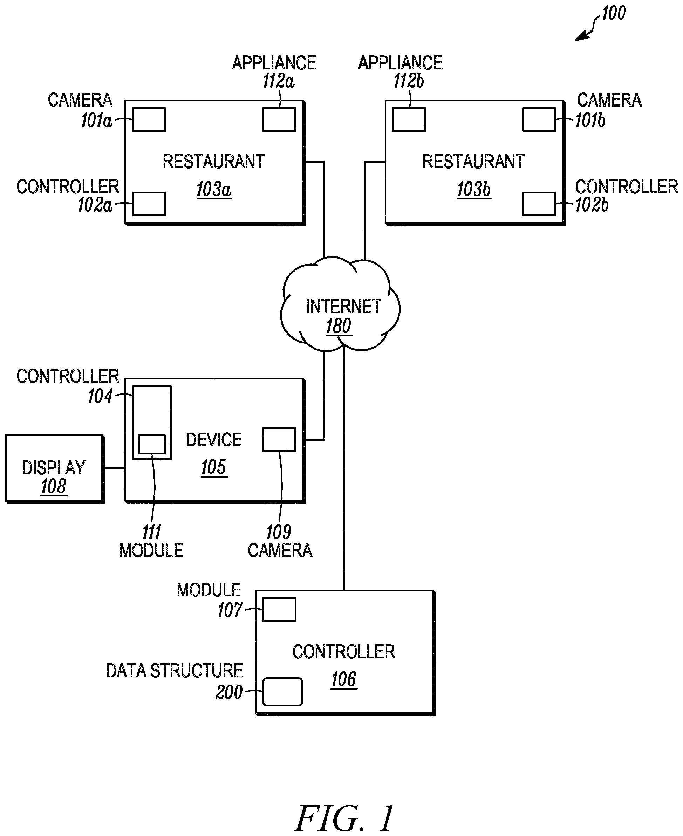

FIG. 1 is a block diagram that illustrates an example system 100 for facilitating dining at an eating establishment, according to an embodiment. In an embodiment, the system 100 includes a network of eating establishments, such as a network of restaurants 103a, 103b that are connected through a network (e.g. internet 180). In one embodiment, each restaurant 103 includes a controller 102 that is communicatively coupled with the network and a camera 101 (e.g. smartphone camera) that is communicatively coupled with the controller 102. Although two restaurants are depicted in FIG. 1, more than two restaurants can be included in the network. In an example embodiment, each restaurant 103 includes one or more appliances 112 that are used to prepare a menu item or dish (e.g. kitchen appliance such as stove, oven, microwave, etc.).

In an embodiment, the system 100 also includes a device 105 (e.g. smartphone) with a camera 109 and a display 108, where the device 105 is also communicatively coupled to the network (e.g. internet 180). In an example embodiment, the device 105 includes a controller 104 that is used to transmit one or more signals between the camera 109, controller 104 and display 108. Although FIG. 1 depicts one device 105, the system 100 is not limited to one device 105 and includes multiple devices 105 simultaneously used by multiple users.

In an embodiment, the system 100 also includes a controller 106 that is communicatively coupled to the network (e.g. internet 180) and includes a database (e.g. data structure 200) to store data received from the network.

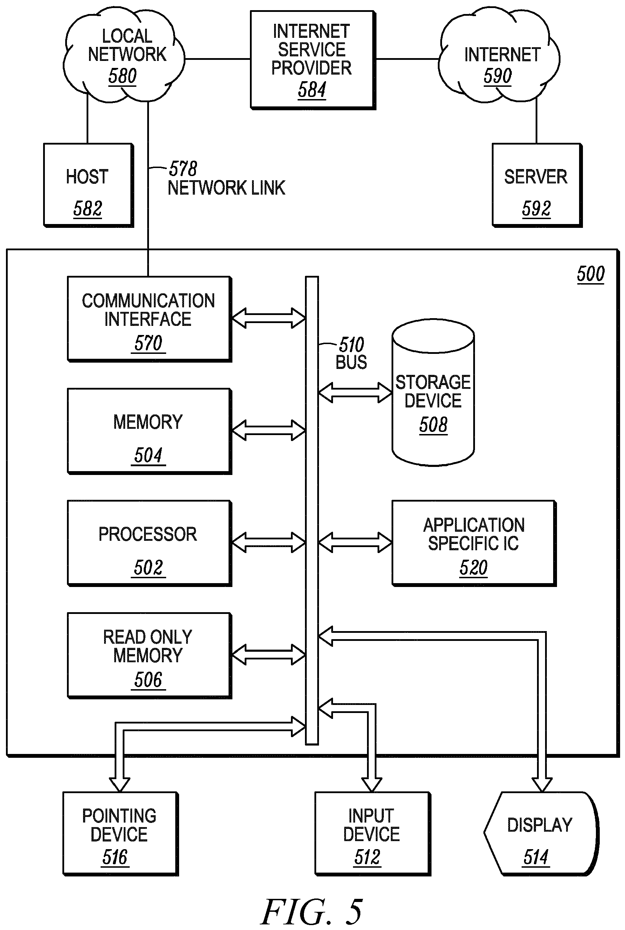

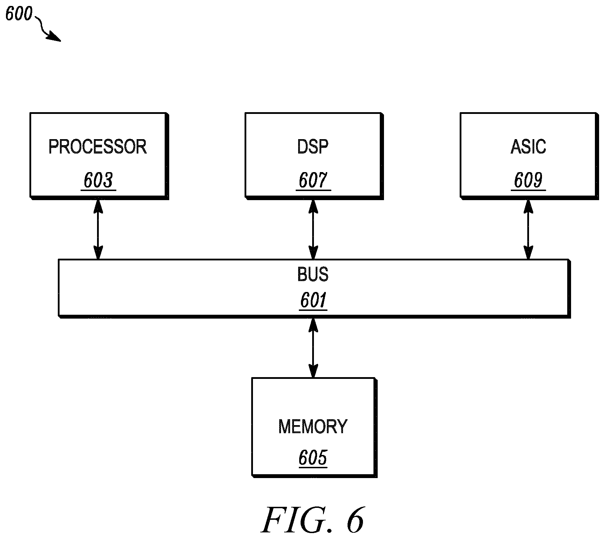

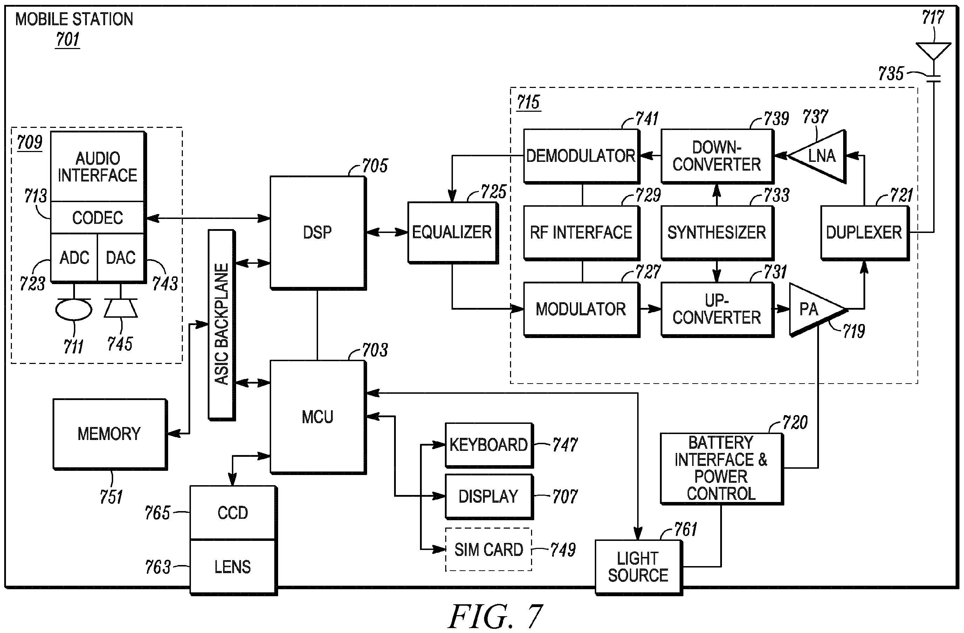

In various embodiments, the controllers 104, 106 comprise one or more general purpose computer systems, as depicted in FIG. 5 or one or more chip sets as depicted in FIG. 6 or one or more mobile terminals as depicted in FIG. 7.

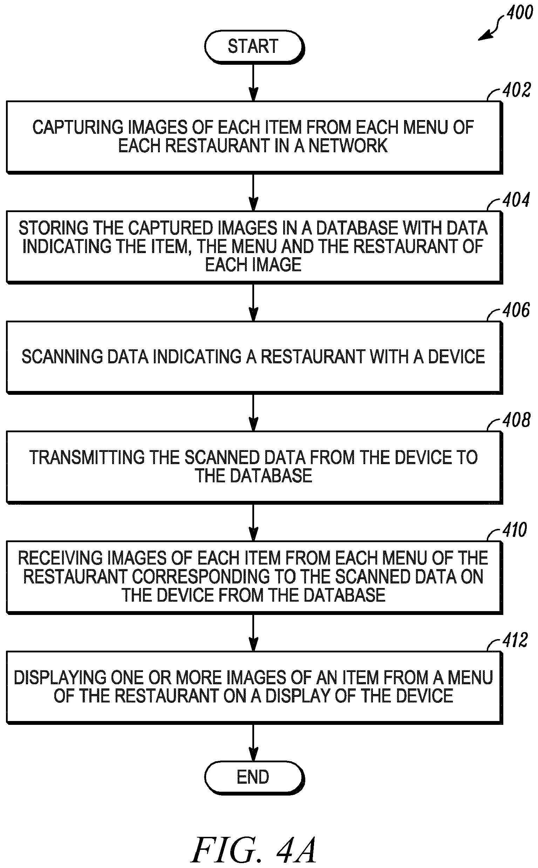

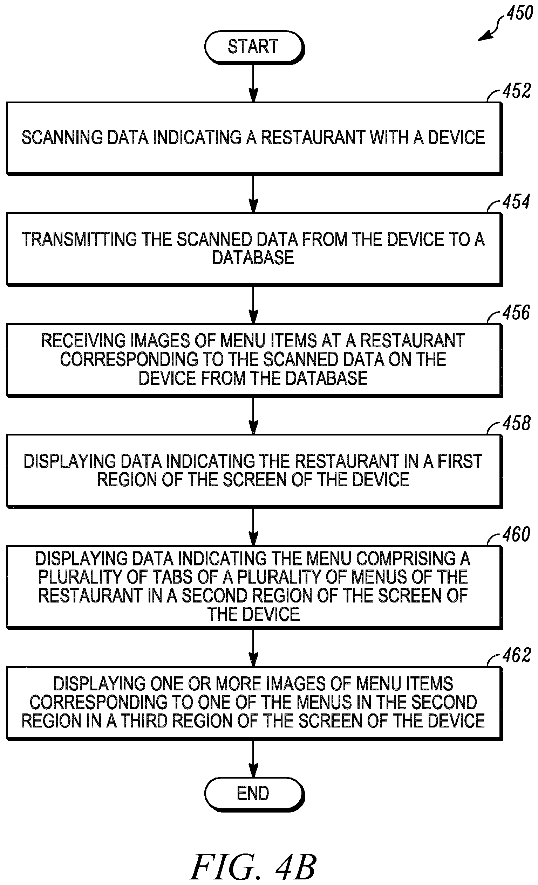

Additionally, in some embodiments the controller 106 includes a module 107 to perform one or more steps of a method described below with reference to FIG. 4A. In other embodiments, the controller 104 includes the module 111 to perform one or more steps of a method described below with reference to FIG. 4B.

In an embodiment, each camera 101 at each restaurant 103 is used to capture one or more images of each item or dish on each menu of the restaurant 103. In one embodiment, each item or dish is prepared in the manner that it is served to the customer. In one example embodiment, an appliance 112 at the restaurant 103 is used to cook or prepare the item or dish. After the item or dish is prepared, a staff member (e.g. chef or photographer) at the restaurant 103 uses the camera 101 to capture one or more images of the item or dish. This is repeated for each dish or item for each menu of the restaurant 103.

The images are then transmitted from the camera 101 to the controller 102 (e.g. a computer located the restaurant or a smartphone with the camera). In an embodiment, each image is stored in a memory of the controller 102 along with data indicating the item, the menu and the restaurant. In an example embodiment, the data indicating the item is a name (e.g. "Warm & Nutty Cinnamon Quinoa") and/or a written description of the item (e.g. one or more sentences describing the item, in one or more foreign languages) and/or a food category or allergy information of the item (e.g. vegan, vegetarian, non-vegetarian, lactose free, etc.) and/or a price of the item (e.g. $20) and/or rating information of the item (e.g. 4.3 stars, 40 reviews) and/or nutritional information of the item (e.g. 200 calories, list of ingredients, etc.). In another example embodiment, the data indicating the menu is one or more of Breakfast, Lunch, Dinner, Brunch, Dessert, etc. In another example embodiment, the data indicating restaurant is a QR code or other data such as a name of the restaurant.

In an embodiment, each controller 102 transmits the images to the controller 106 over the network (e.g. internet 180). In one embodiment, the controller 106 stores each image in the memory of the controller 106, such as in a data structure 200.

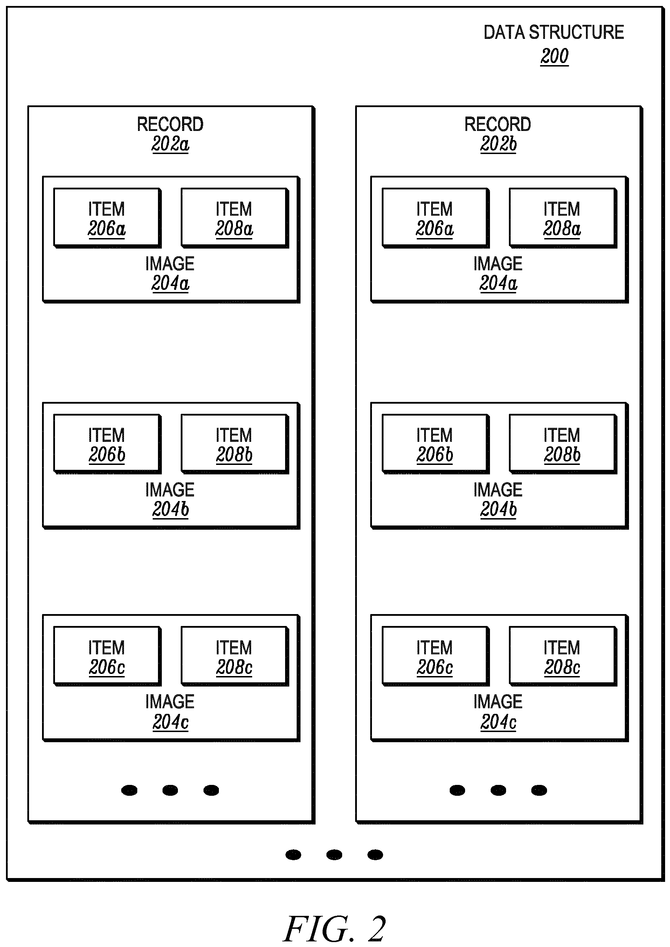

FIG. 2 is a block diagram that illustrates an example data structure 200 used to store image data of the menu items of the eating establishment, according to an embodiment. In an embodiment, the data structure 200 includes a plurality of records 202a, 202b, where each record 202 is provided to store the images for a respective restaurant 103. Although two records 202a, 202b are depicted in FIG. 2, more than two records 202 can be provided in the data structure 200. In an embodiment, the number of records 202 in the data structure 200 corresponds with the number of restaurants 103 in the network. In one embodiment, the record 202 includes a first field to hold data indicating the restaurant 103 (e.g. the QR code) associated with each image. In an example embodiment, the QR code for the restaurant 103a is stored in the record 202a field. In another example embodiment, the QR code for the restaurant 103b is stored in the record 202b field.

In an embodiment, each record 202 includes a plurality of fields 204 to store the images corresponding to each restaurant 103. In an example embodiment, the fields 204a, 204b, 204c of the record 202a store images corresponding to the restaurant 103a and the fields 204a, 204b, 204c of the records 202b store images corresponding to the restaurant 103b. Although FIG. 2 depicts three fields 204 in each record 202, this is merely for illustrative purposes and the record 202 can include more than three fields 204 in order to store more than three images from each restaurant 103. In an example embodiment, the number of fields 204 in each record 202 correspond with the number of images (e.g. of each item from each menu) received from the restaurant 103.

In an embodiment, each image field 204 includes a field 208 for holding data indicating the menu associated with each image in the image field 204. In one embodiment, the field 208 has data indicating a name of the menu (e.g. Breakfast, Lunch, Dinner, Brunch, Dessert, etc., in one or more languages). In an example embodiment, the name of the menu associated with the image field 204a is stored in the field 208a and the name of the menu associated with the image field 204b is stored in the field 208b. and the name of the menu associated with the image field 204c is stored in the field 208c.

In an embodiment, each image field 204 includes a field 206 for holding data indicating the item associated with each image in the image field 204. In one embodiment, the field 206 has data indicating an item name (e.g. "Warm & Nutty Cinnamon Quinoa") and/or a written description of the item (e.g. one or more sentences describing the item, in one or more languages) and/or a food category or allergy information of the item (e.g. vegan, vegetarian, non-vegetarian, lactose free, etc.) and/or a price of the item (e.g. $20) and/or rating information of the item (e.g. 4.3 stars, 40 reviews) and/or nutritional information of the item (e.g. 200 calories, list of ingredients, etc.). In an example embodiment, the item data associated with the image field 204a is stored in the field 206a and the item data associated with the image field 204b is stored in the field 206b. and the item data associated with the image field 204c is stored in the field 206c.

Although processes, equipment, and data structures are depicted in FIG. 1 and FIG. 2 as integral blocks in a particular arrangement for purposes of illustration, in other embodiments one or more processes or data structures, or portions thereof, are arranged in a different manner, on the same or different hosts, in one or more databases, or are omitted, or one or more different processes or data structures are included on the same or different hosts.

Although data structures, messages and fields are depicted in FIG. 2, as integral blocks in a particular order for purposes of illustration, in other embodiments, one or more data structures or messages or fields, or portions thereof, are arranged in a different order, in the same or different number of data structures or databases in one or more hosts or messages, or are omitted, or one or more additional fields are included, or the data structures and messages are changed in some combination of ways.

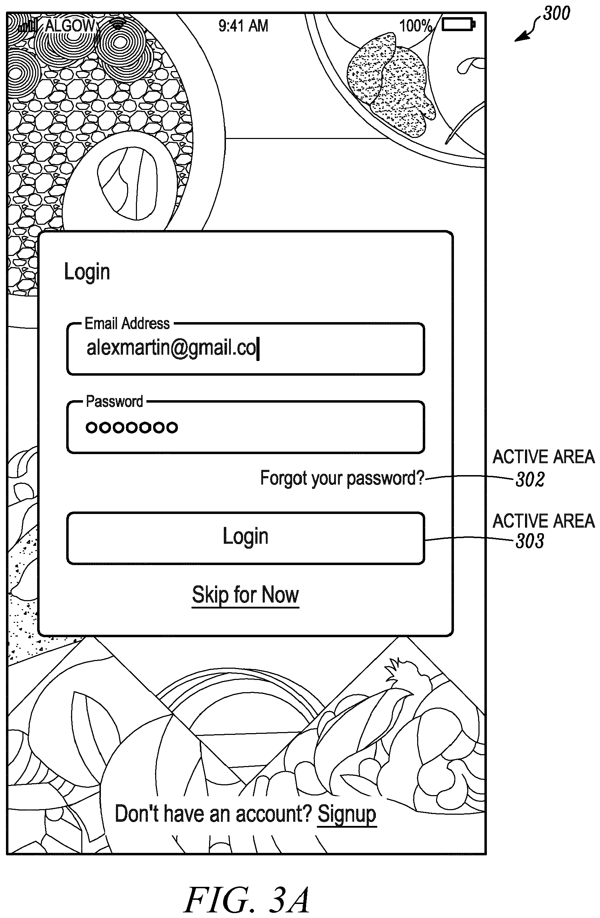

FIG. 3A is an image that illustrates an example of a login view 300 on a display of a software application for facilitating dining at an eating experience, according to an embodiment. In an embodiment, the login view 300 is depicted on the display 108 of the device 105 (e.g. smartphone) that has the software application installed in the controller 104 as the module 111. In one embodiment, the software application is employed by a user who wants to view images of one or more menu items and/or a written description in a preferred language of one or more menu items prior to placing an dine-in, pick-up or delivery order for one or more menu items. In some embodiments, the user is located at the eating establishment and is deciding which menu item to order. In another embodiment, the user is not located at the eating establishment and is deciding whether to visit the eating establishment or place a remote pick-up or delivery order from the eating establishment. It should be noted that FIGS. 3A-3L merely show example embodiments of one software application utilized on a device. Although the software application mentions the use of scanning QR code to identify the restaurant 103, the embodiments of the invention is not limited to scanning QR code and includes any medium or indicia than can be scanned to identify the restaurant 103 or eating establishment.

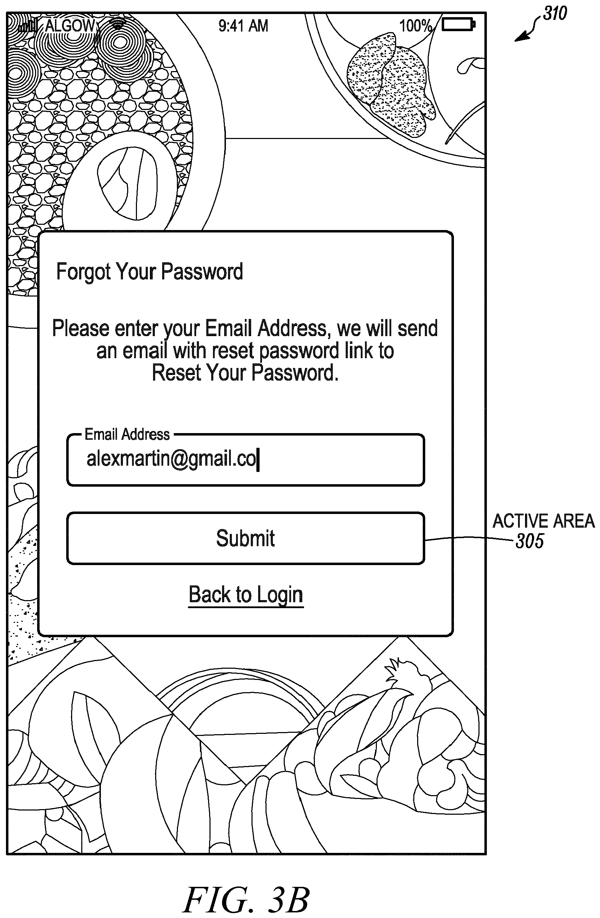

In an embodiment, the login view 300 depicts an input entry for user information (e.g. email address) and a password to log into the application. In an example embodiment, after typing in the email address and password, the user selects the active area 303 to log into the software application. If the user forgets their password, the user selects the active area 302. FIG. 3B is an image that illustrates an example of a lost password view 310 on the display of the software application of FIG. 3A, according to an embodiment. The lost password view 310 is provided on the display upon the user selecting the active area 302 in the login view 300. The user can then enter their email address and select the active area 305. The user will then receive an automatic email to reset their password.

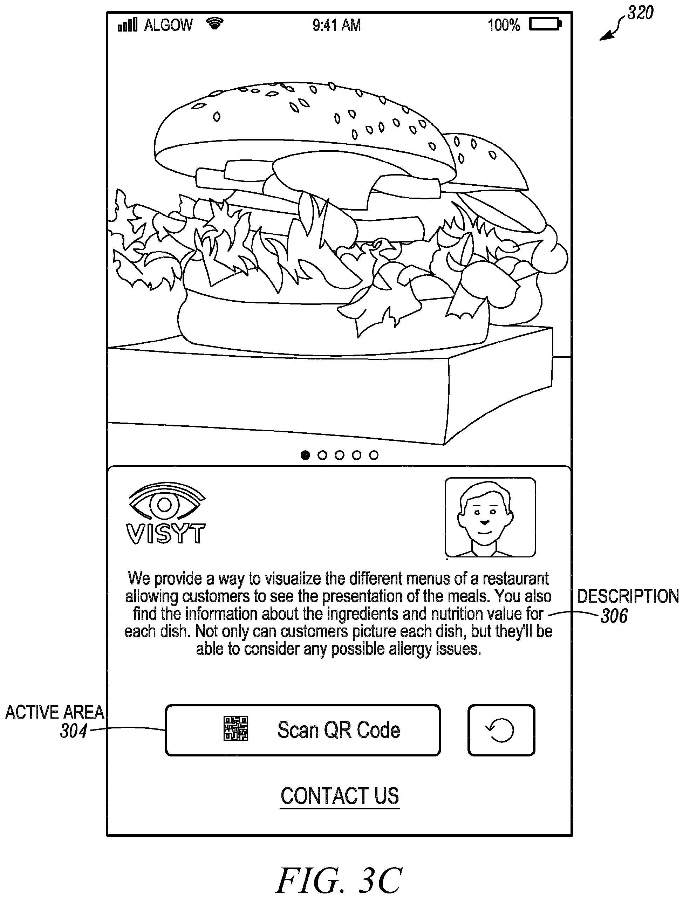

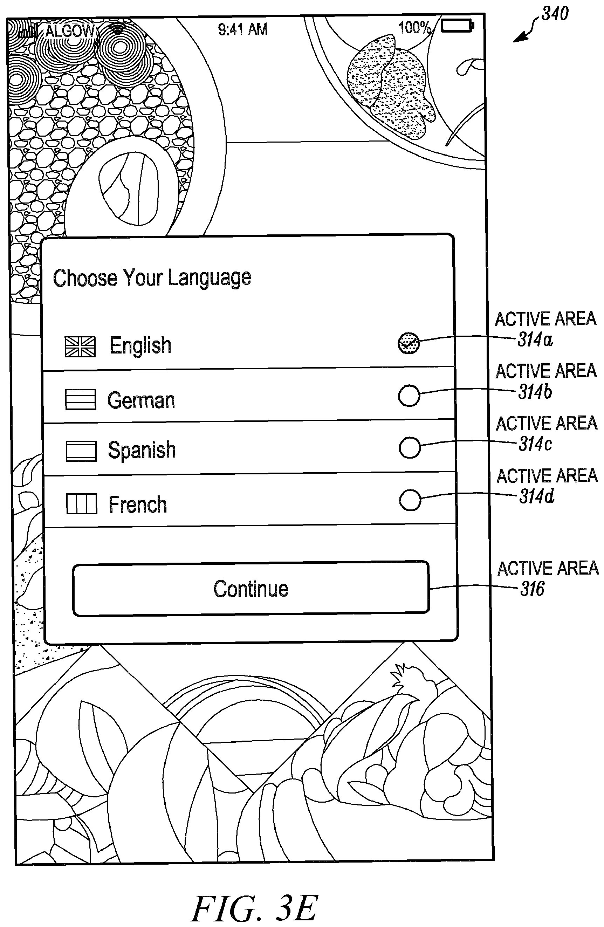

FIG. 3C is an image that illustrates an example of a scan QR code view 320 on the display of the software application of FIG. 3A, according to an embodiment. In an embodiment, the scan QR code view 320 is provided on the display after the user selects the active area 303 in the login view 300 to log into their account. In an embodiment, the scan QR code view 320 includes a written description 306 of the application (e.g. in a designated language selected in the view 340 of FIG. 3E). In an embodiment, an active area 304 is provided on the display and when selected activates a scanner (e.g. camera 109) of the device to scan a QR code of a restaurant 103.

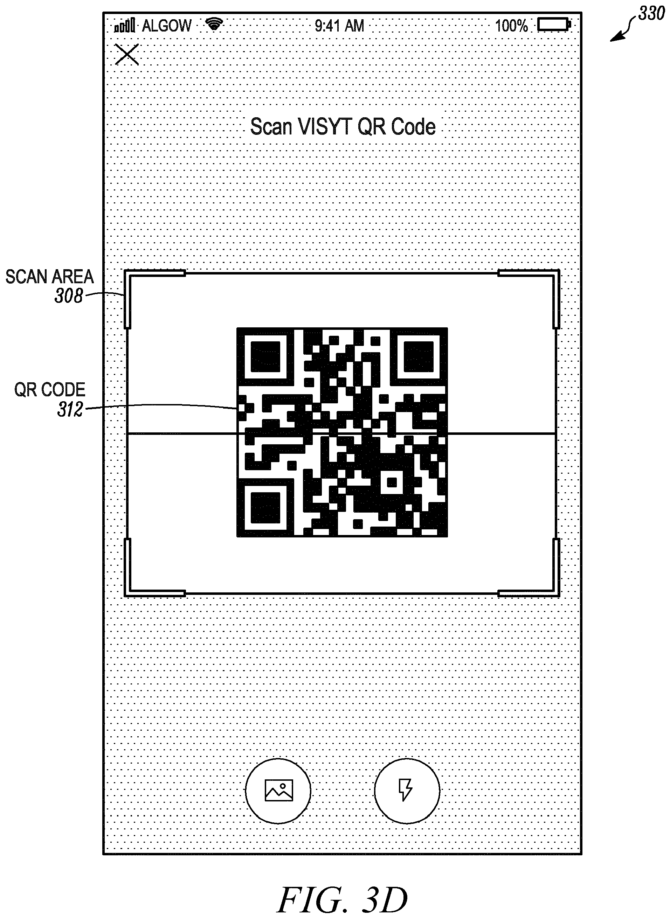

FIG. 3D is an image that illustrates an example of a QR code scan view 330 that includes a QR code scan area 308 on the display of the software application of FIG. 3A, according to an embodiment. In an embodiment, upon the user selecting the active area 304 in the view 320 of FIG. 3C, the scan area 308 is provided on the display and the user moves the scanner (e.g. camera 109) until the QR code 312 of the restaurant 103 is aligned in the scan area 308. In some embodiments, the QR code 312 is scanned as the user is physically located at the restaurant 103 corresponding to the QR code 312. In other embodiments, the QR code 312 is scanned as the user is physically located at a location other than the restaurant 103 (e.g. the user is located at a remote location and is scanning the QR code 312 on a website, menu or other medium with the QR code 312).

FIG. 3E is an image that illustrates an example of a language selection view 340 on the display of the software application of FIG. 3A, according to an embodiment. In an embodiment, after logging into the software application, the language selection view 340 permits the user to select a preferred language in which the item names, item descriptions, etc. (e.g. data from the field 206 for each image 204) is provided on the display. In one embodiment, the language selection view 340 depicts a plurality of languages (e.g. English, German, Spanish, French) with a plurality of respective active areas 314a, 314b, 314c, 314d. In one example embodiment, the user selects the active area 314a corresponding to the preferred language (e.g. English) in which the item name, item description, nutritional information, etc. will be provided on the display during use of the software application. After selecting the active area 314 corresponding to the preferred language, the user selects the active area 316. Although FIG. 3E depicts four active areas for four languages, the language selection view 340 is not limited to four languages and can include less or more than four languages.

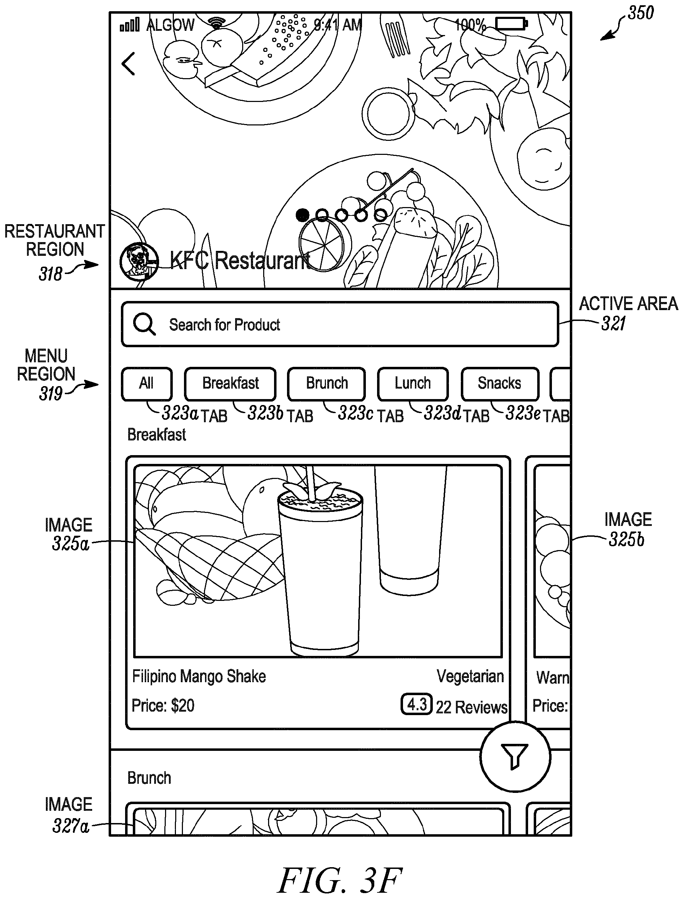

FIG. 3F is an image that illustrates an example of an eating establishment view 350 on the display of the software application of FIG. 3A, according to an embodiment. In an embodiment, the eating establishment view 350 is provided on the display when the QR code 312 of an eating establishment is scanned in the scan area 308 in the QR code scan view 330. In an example embodiment, the camera 109 of the device 105 (e.g. camera of a smartphone) is used to scan the QR code 312 and transmits a signal via the network (e.g. internet 180) to the controller 106 with the scanned QR code 312. In response to receiving this signal, the controller 106 compares the received QR code 312 with the plurality of records 202 to determine which record 202 corresponds to the received QR code 312. The controller 106 then transmits the identified record 202 from the data structure 200 corresponding to the eating establishment with the QR code 312 to the device 105 over the network (e.g. internet 180). In this example embodiment, the display 108 of the device 105 then outputs one or more fields in the record 202 received from the controller 106 over the network. In another embodiment, the eating establishment view 350 is provided on the display when a user manually chooses the eating establishment from list of eating establishments (e.g. after a search query or a list of most recent visited establishments or a list of nearby establishments on a map, etc.).

In an embodiment, the eating establishment view 350 includes a region 318 to display information pertaining to the eating establishment (e.g. name, logo, etc.). In an example embodiment, the region 318 is a restaurant region 318 that displays a name and/or a logo of the restaurant. In an example embodiment, the region 318 of the display provides data from the first field of the record 202 that indicates the name of the restaurant, the logo of the restaurant, etc.

In an embodiment, the eating establishment view 350 includes a menu region 319 that includes multiple tabs 323 of each menu offered by the eating establishment (e.g. restaurant). In one embodiment, the menu region 319 displays data from the field 208 of the record 202 that indicates the different menus of the restaurant and specifically includes a tab 323 for each menu listed in the field 208 of the record 202. As depicted in FIG. 3F, the tab 323b is selected by the user to only show items from the breakfast menu on the display, the tab 323c is selected by the user to only show items from the brunch menu on the display , the tab 323d is selected by the user to only show items from the lunch menu on the display and the tab 323e is selected by the user to only show items from the snack menu on the display. Additionally, the tab 323a is selected by the user to show all items from all menus on the display.

In an embodiment, the eating establishment view 350 includes an active area 321 with a search query where a user can type in a search term to find one or more items among those items offered by the restaurant. In an embodiment, upon the user entering a search term in the active area 321, the controller 104 searches the names of the items (from the field 206 of the record 202 received from the controller 106) and displays one or more items with matching or near matching names.

In an embodiment, the eating establishment view 350 includes a plurality of images 325a, 325b for a plurality of items in a first row sorted according to a first menu (e.g. Breakfast) and a plurality of images 327a, 327b for a plurality of items in a second row sorted according to a second menu (e.g. Brunch). In one embodiment, the images 325, 327 are obtained from the fields 204 of the record 202. In an example embodiment, the images 325 for the first menu are obtained based on those images from the field 204 with the same first menu item field 208a and the images 327 for the second menu are obtained based on those images from the field 204 with the same second menu item field 208b. Additionally, in one embodiment, each image 325, 327 is accompanied by item data (e.g. from the field 206 of the record 202) such as the name of the item, the price of the item, the food category of the item and/or rating information of the item.

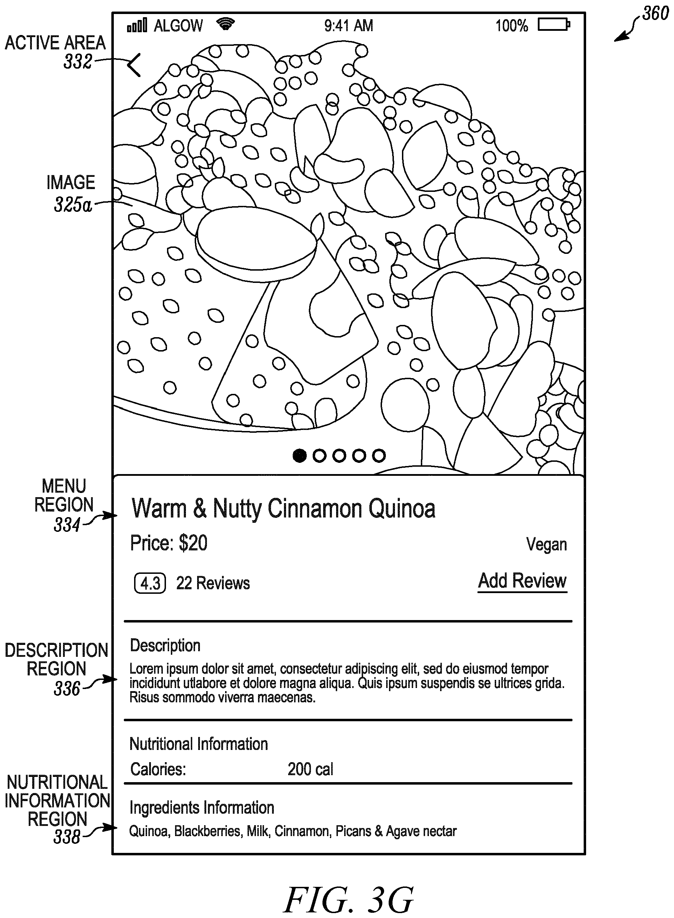

FIG. 3G is an image that illustrates an example of an item view 360 on the display of the software application of FIG. 3A, according to an embodiment. In an embodiment, the item view 360 is provided on the display upon a user selecting one of the items from the eating establishment view 350 of FIG. 3F. In an embodiment, the item view 360 includes an image 325a of the item which is provided by the field 204 of the record 202. In another embodiment, the item view 360 includes an active area 332 so a user can toggle between multiple images (e.g. obtained from the field 204 for items with the same item field 206) so that a user can view multiple images of the item. In an example embodiment, the active area 332 permits the user to advantageously view the item from multiple angles. In another embodiment, the item view 360 includes an item region 334 that includes item data (e.g. from the field 206 of the record 202) such as the name of the item, the food category or allergy information of the item and/or rating information of the item. In another embodiment, the item view 360 includes a description region 336 that includes a written description (e.g. less than 100 words) of the item in a selected language based on the language selection view 340. In an example embodiment, the written description in the designated language is obtained from the item field 206 of the record 202. In another embodiment, the item view 360 includes the nutritional information region 338 that provides nutritional information (e.g. ingredients, calories, amount of fat, carbohydrates, protein, etc.) based on data from the item field 206 of the record 202.

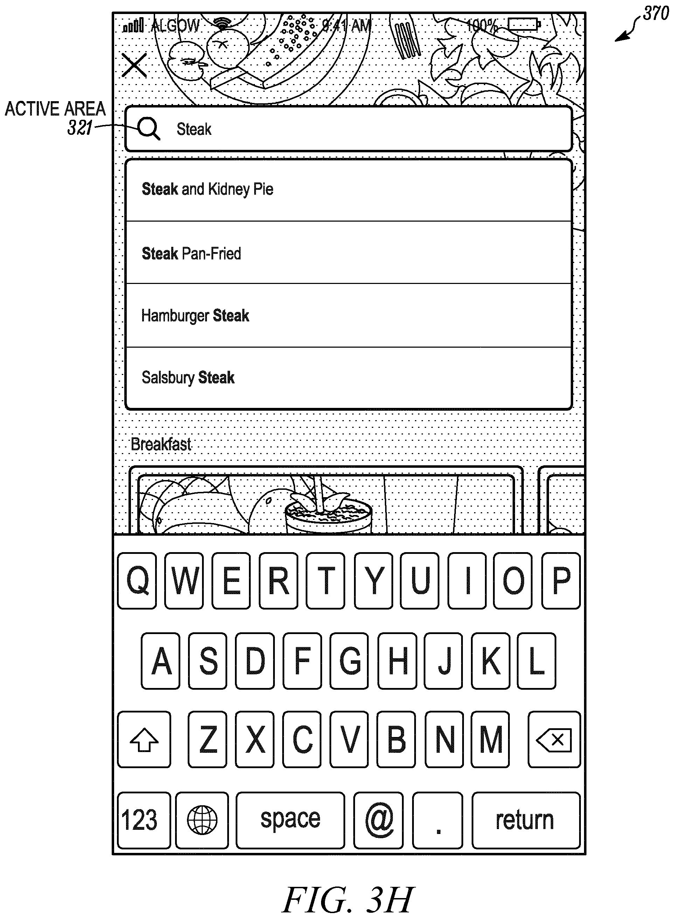

FIG. 3H is an image that illustrates an example of a search view 370 on the display of the software application of FIG. 3A, according to an embodiment. In an embodiment, the search view 370 provides the active area 321 for the user to enter one or more terms to find an item at the eating establishment with those terms or close to those terms. After entering the one or more terms the items whose name matches the terms appears in a list underneath the active area 321. In an example embodiment, after the user types in the one or more terms, the controller 104 searches the names of the items (e.g. from the field 206 of the record 202). In a further example embodiment, upon selecting one of the items in the list below the active area 321, the display outputs the item view 360 corresponding with that selected item.

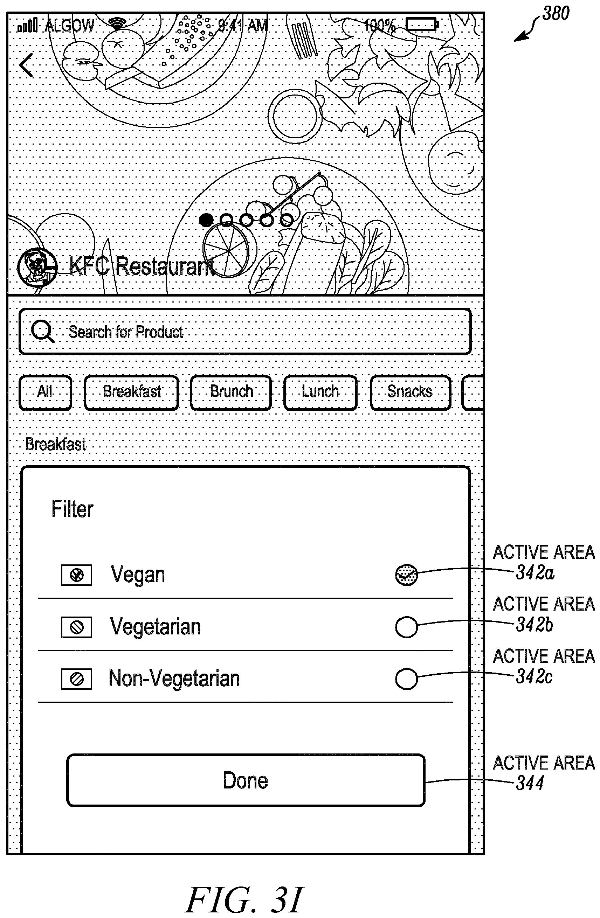

FIG. 3I is an image that illustrates an example of a food category selection view 380 on the display of the software application of FIG. 3A, according to an embodiment. In an embodiment, the food category selection view 380 includes a plurality of active areas 342a, 342b, 342c corresponding to a respective plurality of food categories (e.g. vegan, vegetarian, non-vegetarian). The food categories are not limited to those listed in FIG. 3I. Upon the user selecting one of the active areas 342a, the user then selects the active area 344 which then filters the items displayed in the eating establishment view 350 to only include those items corresponding to the food category of the selected active area 342a.

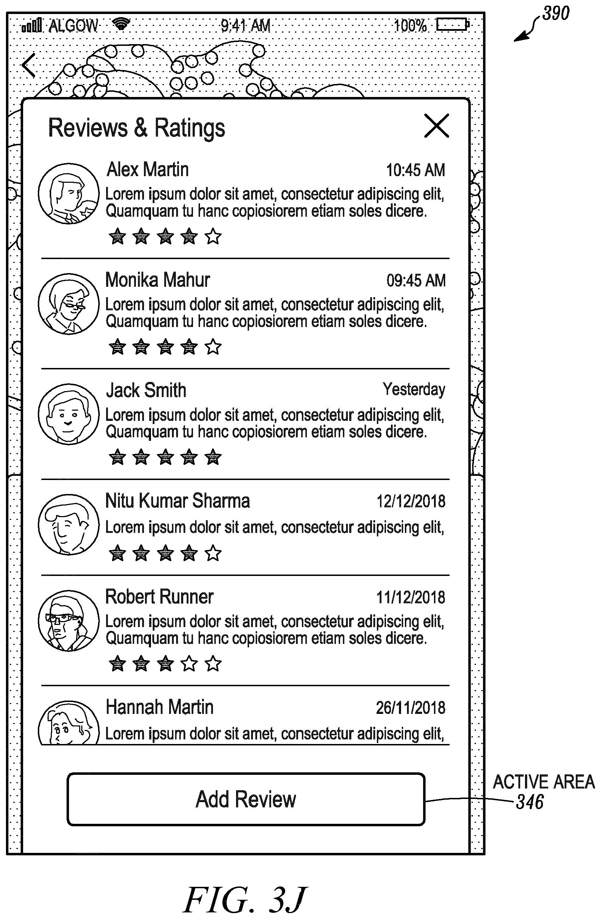

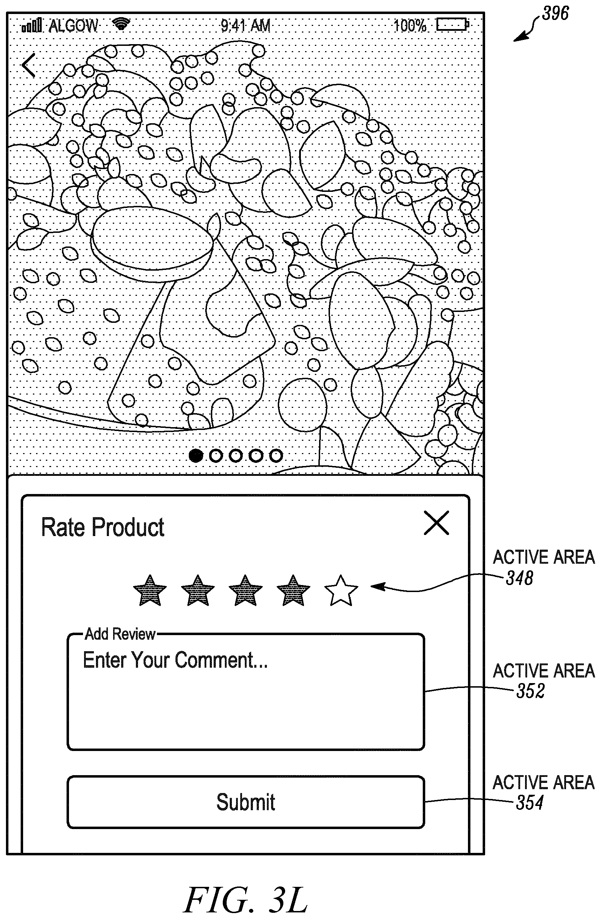

FIG. 3J is an image that illustrates an example of a review and rating view 390 on the display of the software application of FIG. 3A, according to an embodiment. In an embodiment, the view 390 includes a list of a plurality of combined ratings and reviews of patrons who recently visited the eating establishment. In some embodiments, the ratings and reviews are for the eating establishment. In other embodiments, the ratings and reviews are for a particular item at the eating establishment. In an embodiment, the view 390 includes an active area 346 which the user can select to add a review after the user has eaten an item at the eating establishment. In an embodiment, upon selecting the active area, the display provides the rate product view 396 of FIG. 3L. In an embodiment, the view 396 includes an active area 348 where the user can enter a star rating (e.g. 1-5 stars) for the item and another active area 352 where a user can enter a written review of the item. After the user enters the star rating and/or the written review, the user selects the active area 354 so that a signal including data indicating the star and written description of the review is transmitted from the device 105 to the controller 106 over the network (e.g. internet 180) and is stored in the item field 206 for the record 202 corresponding to the eating establishment.

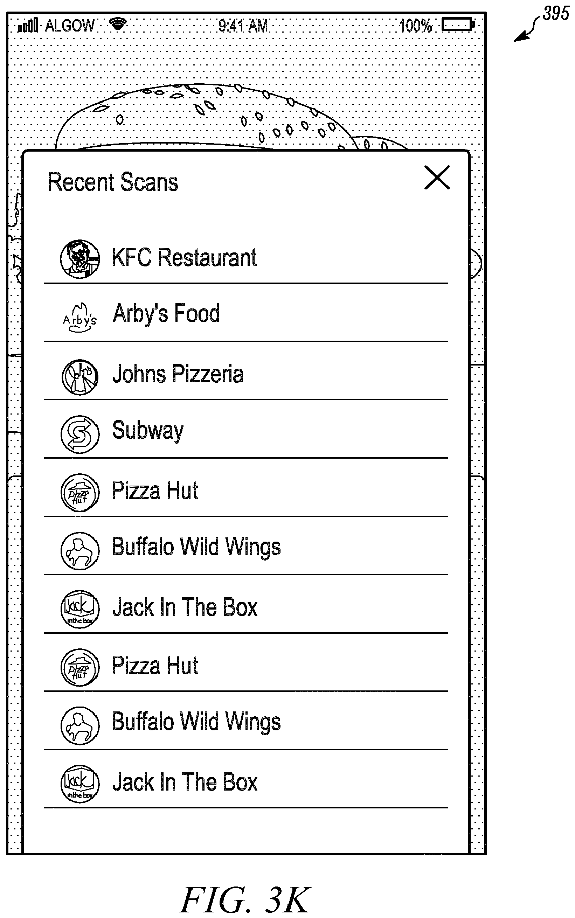

FIG. 3K is an image that illustrates an example of a recent scan view 395 on the display of the software application of FIG. 3A, according to an embodiment. In an embodiment, the recent scan view 396 includes a list of eating establishments that the user of the device 105 has recently scanned (e.g. QR code 312) with the camera 109. In an embodiment, the recent scan view 395 conveniently allows the user to select one of the recently scanned eating establishments, to alleviate the need of the user to re-scan the QR code 312 of that eating establishment. Upon selecting an eating establishment, the eating establishment view 350 is provided on the display.

Although steps are depicted in FIGS. 4A-4B, as integral steps in a particular order for purposes of illustration, in other embodiments, one or more steps, or portions thereof, are performed in a different order, or overlapping in time, in series or in parallel, or are omitted, or one or more additional steps are added, or the method is changed in some combination of ways.

FIG. 4A is a flow diagram that illustrates an example of a method 400 for capturing and storing image data of menu items of a plurality of eating establishments, according to an embodiment. In step 402, images of each item from each menu of each eating establishment in a network is provided. In an embodiment, the eating establishment is a restaurant. In an embodiment, in step 402 each item is prepared at each restaurant 103 in a manner that it appears when it is served to a patron of the restaurant. In one embodiment, each item is prepared at the restaurant 103 using an appliance 112 such as a kitchen appliance (e.g. stove, oven, microwave, etc.). In an example embodiment, staff at the restaurant (e.g. chef) prepare each item using the appliance 112. In an embodiment, in step 402 after each item is prepared, a staff member at the restaurant 103 uses a camera 101 to capture one or more images of each item. The images of each item are stored in a memory of the controller 102 at each restaurant with data indicating the item, the menu and the restaurant. In an example embodiment, the data indicating the restaurant is the QR code 312. In an embodiment, step 402 is performed for each item on each menu of the restaurant 103. In another embodiment, step 402 is performed for each restaurant 103a, 103b in the network. Although two restaurants are depicted in FIG. 1, more than two restaurants can be provided in the network.

In step 404, the captured images in step 402 are stored in a database with data indicating the item, the menu and the restaurant of each image. In an embodiment, the captured images from each restaurant 103 are transmitted (e.g. over the internet 180) to the controller 106 and stored in a memory of the controller 106. In an embodiment, the memory of the controller 106 includes the data structure 200. In an embodiment, each controller 102 of each restaurant 103 transmits the captured images from step 402 to the controller 106 and the captured images from each restaurant 103 are stored in a respective record 202 of the data structure 200. In an embodiment, the images are stored in the image field 204 of the record 202, so that a plurality of images are stored in a plurality of images fields 204a, 204b, 204c of the record 202. In another embodiment, the data indicating each item (e.g. name of the item, written description of the item in multiple languages, rating information, nutritional information, etc.) are stored in the field 206 for each image field 204. In another embodiment, the data indicating the menu (e.g. Breakfast, Brunch, Lunch, etc.) is stored in the field 208 for each image field 204. In another embodiment, in step 404 the images from the first restaurant 103a (e.g. from controller 102a) are stored in a first record 202a and the images from the second restaurant 103b (e.g. from controller 102b) are stored in a second record 202b. As with step 402, although the depicted data structure 200 of FIG. 2 shows two records 202a, 202b in other embodiments, more than two records are provided so that the number of records is equal to or greater than the number of restaurants 103 in the network.

In step 406, data indicating an eating establishment is scanned by a device. In an embodiment, the data indicating the eating establishment is a QR code. In another embodiment, the device is the camera 109 of the device 105 (e.g. smartphone). In an embodiment, in step 406 the scan QR Code view 320 is provided on the display 108 after which the user selects the active area 304 and the QR code scan view 330 is provided on the display 108 so that camera 109 can be used to scan the QR code 312 by positioning the QR code 312 in the scan area 308 of the camera 109.

In step 408, after the data indicating the eating establishment is scanned in step 406, the scanned data is transmitted from the device to the database. In an embodiment, in step 408 after the camera 109 scans the QR code 312 of the restaurant in step 406, the device 105 transmits a signal with the QR code 312 over the network (e.g. internet 180) to the controller 106. In an embodiment, the controller 106 compares the data indicating the eating establishment (e.g. QR code 312) in the signal with each record 202 in the data structure 200 to determine the record 202 that corresponds with the received QR code 312. In an embodiment, the controller 106 then transmits a signal to the device 105 over the network (e.g. internet 180) with the record 202 that corresponds with the received QR code 312.

In step 410, the images transmitted from the controller 106 are received at the device 105. In an embodiment, the controller 104 of the device 105 receives the images which are stored based on the data indicating the item (field 206), the data indicating the menu (field 208) and the image (field 204). In an embodiment, in step 410 the device 105 receives the images for each menu item at the eating establishment corresponding to the scanned QR code 312.

In step 412, the display 108 of the device 105 outputs one or more of the images received in step 410. In an embodiment, step 412 is based on the software application discussed in FIGS. 3A-3L that can be implemented on the device 105 (e.g. smartphone).

FIG. 4B is a flow diagram that illustrates an example of a method 450 for displaying images of menu items from one of a plurality of eating establishments, according to an embodiment. Steps 452, 454, 456 are similar to respective steps 406, 408, 410 of the method 400.

In an embodiment, steps 458, 460, 462 are one embodiment of step 412. In an embodiment, step 458 is displaying data indicating the restaurant in a first region of the display of the device. In an embodiment, step 458 is depicted in the eating establishment view 350 of FIG. 3F which depicts the data indicating the restaurant (e.g. name, logo) in the restaurant region 318 of the display 108 of the device 105. In an embodiment, step 460 is displaying data indicating the menu including a plurality of tabs of a plurality of menus of the restaurant in a second region of the display of the device. In an embodiment, step 460 is depicted in the eating establishment view 350 of FIG. 3F which depicts the data indicating the menu (e.g. All, Breakfast, Brunch, Lunch, Snacks, etc.) including a plurality of tabs 323a, 323b, 323c, 323d, 323e of the plurality of menus in a menu region 319 of the display 108 of the device 105.

In an embodiment, step 462 is displaying one or more images corresponding to one of the menus in the second region in a third region of the display of the device. In an embodiment, step 462 is depicted in the eating establishment view 350 of FIG. 3F which depicts the images 325a, 325b corresponding to one of the menus (e.g. Breakfast) in a third region of the display 108 and images 327a, 327b corresponding to one of the menus (e.g. Brunch) in a fourth region of the display.

### 2. Hardware Overview

FIG. 5 is a block diagram that illustrates a computer system 500 upon which an embodiment of the invention may be implemented. Computer system 500 includes a communication mechanism such as a bus 510 for passing information between other internal and external components of the computer system 500. Information is represented as physical signals of a measurable phenomenon, typically electric voltages, but including, in other embodiments, such phenomena as magnetic, electromagnetic, pressure, chemical, molecular atomic and quantum interactions. For example, north and south magnetic fields, or a zero and non-zero electric voltage, represent two states (0, 1) of a binary digit (bit). Other phenomena can represent digits of a higher base. A superposition of multiple simultaneous quantum states before measurement represents a quantum bit (qubit). A sequence of one or more digits constitutes digital data that is used to represent a number or code for a character. In some embodiments, information called analog data is represented by a near continuum of measurable values within a particular range. Computer system 500, or a portion thereof, constitutes a means for performing one or more steps of one or more methods described herein.

A sequence of binary digits constitutes digital data that is used to represent a number or code for a character. A bus 510 includes many parallel conductors of information so that information is transferred quickly among devices coupled to the bus 510. One or more processors 502 for processing information are coupled with the bus 510. A processor 502 performs a set of operations on information. The set of operations include bringing information in from the bus 510 and placing information on the bus 510. The set of operations also typically include comparing two or more units of information, shifting positions of units of information, and combining two or more units of information, such as by addition or multiplication. A sequence of operations to be executed by the processor 502 constitutes computer instructions.

Computer system 500 also includes a memory 504 coupled to bus 510. The memory 504, such as a random access memory (RAM) or other dynamic storage device, stores information including computer instructions. Dynamic memory allows information stored therein to be changed by the computer system 500. RAM allows a unit of information stored at a location called a memory address to be stored and retrieved independently of information at neighboring addresses. The memory 504 is also used by the processor 502 to store temporary values during execution of computer instructions. The computer system 500 also includes a read only memory (ROM) 506 or other static storage device coupled to the bus 510 for storing static information, including instructions, that is not changed by the computer system 500. Also coupled to bus 510 is a non-volatile (persistent) storage device 508, such as a magnetic disk or optical disk, for storing information, including instructions, that persists even when the computer system 500 is turned off or otherwise loses power.

Information, including instructions, is provided to the bus 510 for use by the processor from an external input device 512, such as a keyboard containing alphanumeric keys operated by a human user, or a sensor. A sensor detects conditions in its vicinity and transforms those detections into signals compatible with the signals used to represent information in computer system 500. Other external devices coupled to bus 510, used primarily for interacting with humans, include a display device 514, such as a cathode ray tube (CRT) or a liquid crystal display (LCD), for presenting images, and a pointing device 516, such as a mouse or a trackball or cursor direction keys, for controlling a position of a small cursor image presented on the display 514 and issuing commands associated with graphical elements presented on the display 514.

In the illustrated embodiment, special purpose hardware, such as an application specific integrated circuit (IC) 520, is coupled to bus 510. The special purpose hardware is configured to perform operations not performed by processor 502 quickly enough for special purposes. Examples of application specific ICs include graphics accelerator cards for generating images for display 514, cryptographic boards for encrypting and decrypting messages sent over a network, speech recognition, and interfaces to special external devices, such as robotic arms and medical scanning equipment that repeatedly perform some complex sequence of operations that are more efficiently implemented in hardware.

Computer system 500 also includes one or more instances of a communications interface 570 coupled to bus 510. Communication interface 570 provides a two-way communication coupling to a variety of external devices that operate with their own processors, such as printers, scanners and external disks. In general, the coupling is with a network link 578 that is connected to a local network 580 to which a variety of external devices with their own processors are connected. For example, communication interface 570 may be a parallel port or a serial port or a universal serial bus (USB) port on a personal computer. In some embodiments, communications interface 570 is an integrated services digital network (ISDN) card or a digital subscriber line (DSL) card or a telephone modem that provides an information communication connection to a corresponding type of telephone line. In some embodiments, a communication interface 570 is a cable modem that converts signals on bus 510 into signals for a communication connection over a coaxial cable or into optical signals for a communication connection over a fiber optic cable. As another example, communications interface 570 may be a local area network (LAN) card to provide a data communication connection to a compatible LAN, such as Ethernet. Wireless links may also be implemented. Carrier waves, such as acoustic waves and electromagnetic waves, including radio, optical and infrared waves travel through space without wires or cables. Signals include man-made variations in amplitude, frequency, phase, polarization or other physical properties of carrier waves. For wireless links, the communications interface 570 sends and receives electrical, acoustic or electromagnetic signals, including infrared and optical signals, that carry information streams, such as digital data.

The term computer-readable medium is used herein to refer to any medium that participates in providing information to processor 502, including instructions for execution. Such a medium may take many forms, including, but not limited to, non-volatile media, volatile media and transmission media. Non-volatile media include, for example, optical or magnetic disks, such as storage device 508. Volatile media include, for example, dynamic memory 504. Transmission media include, for example, coaxial cables, copper wire, fiber optic cables, and waves that travel through space without wires or cables, such as acoustic waves and electromagnetic waves, including radio, optical and infrared waves. The term computer-readable storage medium is used herein to refer to any medium that participates in providing information to processor 502, except for transmission media.

Common forms of computer-readable media include, for example, a floppy disk, a flexible disk, a hard disk, a magnetic tape, or any other magnetic medium, a compact disk ROM (CD-ROM), a digital video disk (DVD) or any other optical medium, punch cards, paper tape, or any other physical medium with patterns of holes, a RAM, a programmable ROM (PROM), an erasable PROM (EPROM), a FLASH-EPROM, or any other memory chip or cartridge, a carrier wave, or any other medium from which a computer can read. The term non-transitory computer-readable storage medium is used herein to refer to any medium that participates in providing information to processor 502, except for carrier waves and other signals.

Logic encoded in one or more tangible media includes one or both of processor instructions on a computer-readable storage media and special purpose hardware, such as ASIC *520.

Network link 578 typically provides information communication through one or more networks to other devices that use or process the information. For example, network link 578 may provide a connection through local network 580 to a host computer 582 or to equipment 584 operated by an Internet Service Provider (ISP). ISP equipment 584 in turn provides data communication services through the public, world-wide packet-switching communication network of networks now commonly referred to as the Internet 590. A computer called a server 592 connected to the Internet provides a service in response to information received over the Internet. For example, server 592 provides information representing video data for presentation at display 514.

The invention is related to the use of computer system 500 for implementing the techniques described herein. According to one embodiment of the invention, those techniques are performed by computer system 500 in response to processor 502 executing one or more sequences of one or more instructions contained in memory 504. Such instructions, also called software and program code, may be read into memory 504 from another computer-readable medium such as storage device 508. Execution of the sequences of instructions contained in memory 504 causes processor 502 to perform the method steps described herein. In alternative embodiments, hardware, such as application specific integrated circuit 520, may be used in place of or in combination with software to implement the invention. Thus, embodiments of the invention are not limited to any specific combination of hardware and software.

The signals transmitted over network link 578 and other networks through communications interface 570, carry information to and from computer system 500. Computer system 500 can send and receive information, including program code, through the networks 580, 590 among others, through network link 578 and communications interface 570. In an example using the Internet 590, a server 592 transmits program code for a particular application, requested by a message sent from computer 500, through Internet 590, ISP equipment 584, local network 580 and communications interface 570. The received code may be executed by processor 502 as it is received, or may be stored in storage device 508 or other non-volatile storage for later execution, or both. In this manner, computer system 500 may obtain application program code in the form of a signal on a carrier wave.

Various forms of computer readable media may be involved in carrying one or more sequence of instructions or data or both to processor 502 for execution. For example, instructions and data may initially be carried on a magnetic disk of a remote computer such as host 582. The remote computer loads the instructions and data into its dynamic memory and sends the instructions and data over a telephone line using a modem. A modem local to the computer system 500 receives the instructions and data on a telephone line and uses an infra-red transmitter to convert the instructions and data to a signal on an infra-red a carrier wave serving as the network link 578. An infrared detector serving as communications interface 570 receives the instructions and data carried in the infrared signal and places information representing the instructions and data onto bus 510. Bus 510 carries the information to memory 504 from which processor 502 retrieves and executes the instructions using some of the data sent with the instructions. The instructions and data received in memory 504 may optionally be stored on storage device 508, either before or after execution by the processor 502.

FIG. 6 illustrates a chip set 600 upon which an embodiment of the invention may be implemented. Chip set 600 is programmed to perform one or more steps of a method described herein and includes, for instance, the processor and memory components described with respect to FIG. *8 incorporated in one or more physical packages (e.g., chips). By way of example, a physical package includes an arrangement of one or more materials, components, and/or wires on a structural assembly (e.g., a baseboard) to provide one or more characteristics such as physical strength, conservation of size, and/or limitation of electrical interaction. It is contemplated that in certain embodiments the chip set can be implemented in a single chip. Chip set 600, or a portion thereof, constitutes a means for performing one or more steps of a method described herein.

In one embodiment, the chip set 600 includes a communication mechanism such as a bus 601 for passing information among the components of the chip set 600. A processor 603 has connectivity to the bus 601 to execute instructions and process information stored in, for example, a memory 605. The processor 603 may include one or more processing cores with each core configured to perform independently. A multi-core processor enables multiprocessing within a single physical package. Examples of a multi-core processor include two, four, eight, or greater numbers of processing cores. Alternatively, or in addition, the processor 603 may include one or more microprocessors configured in tandem via the bus 601 to enable independent execution of instructions, pipelining, and multithreading. The processor 603 may also be accompanied with one or more specialized components to perform certain processing functions and tasks such as one or more digital signal processors (DSP) 607, or one or more application-specific integrated circuits (ASIC) 609. A DSP 607 typically is configured to process real-world signals (e.g., sound) in real time independently of the processor 603. Similarly, an ASIC 609 can be configured to performed specialized functions not easily performed by a general purposed processor. Other specialized components to aid in performing the inventive functions described herein include one or more field programmable gate arrays (FPGA) (not shown), one or more controllers (not shown), or one or more other special-purpose computer chips.

The processor 603 and accompanying components have connectivity to the memory 605 via the bus 601. The memory 605 includes both dynamic memory (e.g., RAM, magnetic disk, writable optical disk, etc.) and static memory (e.g., ROM, CD-ROM, etc.) for storing executable instructions that when executed perform one or more steps of a method described herein. The memory 605 also stores the data associated with or generated by the execution of one or more steps of the methods described herein.

FIG. 7 is a diagram of exemplary components of a mobile terminal 700 (e.g., cell phone handset) for communications, which is capable of operating in the system of FIG. 2C, according to one embodiment. In some embodiments, mobile terminal 701, or a portion thereof, constitutes a means for performing one or more steps described herein. Generally, a radio receiver is often defined in terms of front-end and back-end characteristics. The front-end of the receiver encompasses all of the Radio Frequency (RF) circuitry whereas the back-end encompasses all of the base-band processing circuitry. As used in this application, the term "circuitry" refers to both: (1) hardware-only implementations (such as implementations in only analog and/or digital circuitry), and (2) to combinations of circuitry and software (and/or firmware) (such as, if applicable to the particular context, to a combination of processor(s), including digital signal processor(s), software, and memory(ies) that work together to cause an apparatus, such as a mobile phone or server, to perform various functions). This definition of "circuitry" applies to all uses of this term in this application, including in any claims. As a further example, as used in this application and if applicable to the particular context, the term "circuitry" would also cover an implementation of merely a processor (or multiple processors) and its (or their) accompanying software/or firmware. The term "circuitry" would also cover if applicable to the particular context, for example, a baseband integrated circuit or applications processor integrated circuit in a mobile phone or a similar integrated circuit in a cellular network device or other network devices.

Pertinent internal components of the telephone include a Main Control Unit (MCU) 703, a Digital Signal Processor (DSP) 705, and a receiver/transmitter unit including a microphone gain control unit and a speaker gain control unit. A main display unit 707 provides a display to the user in support of various applications and mobile terminal functions that perform or support the steps as described herein. The display 707 includes display circuitry configured to display at least a portion of a user interface of the mobile terminal (e.g., mobile telephone). Additionally, the display 707 and display circuitry are configured to facilitate user control of at least some functions of the mobile terminal. An audio function circuitry 709 includes a microphone 711 and microphone amplifier that amplifies the speech signal output from the microphone 711. The amplified speech signal output from the microphone 711 is fed to a coder/decoder (CODEC) 713.

A radio section 715 amplifies power and converts frequency in order to communicate with a base station, which is included in a mobile communication system, via antenna 717. The power amplifier (PA) 719 and the transmitter/modulation circuitry are operationally responsive to the MCU 703, with an output from the PA 719 coupled to the duplexer 721 or circulator or antenna switch, as known in the art. The PA 719 also couples to a battery interface and power control unit 720.

In use, a user of mobile terminal 701 speaks into the microphone 711 and his or her voice along with any detected background noise is converted into an analog voltage. The analog voltage is then converted into a digital signal through the Analog to Digital Converter (ADC) 723. The control unit 703 routes the digital signal into the DSP 705 for processing therein, such as speech encoding, channel encoding, encrypting, and interleaving. In one embodiment, the processed voice signals are encoded, by units not separately shown, using a cellular transmission protocol such as enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., microwave access (WiMAX), Long Term Evolution (LTE) networks, code division multiple access (CDMA), wideband code division multiple access (WCDMA), wireless fidelity (Wi-Fi), satellite, and the like, or any combination thereof.

The encoded signals are then routed to an equalizer 725 for compensation of any frequency-dependent impairments that occur during transmission though the air such as phase and amplitude distortion. After equalizing the bit stream, the modulator 727 combines the signal with a RF signal generated in the RF interface 729. The modulator 727 generates a sine wave by way of frequency or phase modulation. In order to prepare the signal for transmission, an up-converter 731 combines the sine wave output from the modulator 727 with another sine wave generated by a synthesizer 733 to achieve the desired frequency of transmission. The signal is then sent through a PA 719 to increase the signal to an appropriate power level. In practical systems, the PA 719 acts as a variable gain amplifier whose gain is controlled by the DSP 705 from information received from a network base station. The signal is then filtered within the duplexer 721 and optionally sent to an antenna coupler 735 to match impedances to provide maximum power transfer. Finally, the signal is transmitted via antenna 717 to a local base station. An automatic gain control (AGC) can be supplied to control the gain of the final stages of the receiver. The signals may be forwarded from there to a remote telephone which may be another cellular telephone, any other mobile phone or a land-line connected to a Public Switched Telephone Network (PSTN), or other telephony networks.

Voice signals transmitted to the mobile terminal 701 are received via antenna 717 and immediately amplified by a low noise amplifier (LNA) 737. A down-converter 739 lowers the carrier frequency while the demodulator 741 strips away the RF leaving only a digital bit stream. The signal then goes through the equalizer 725 and is processed by the DSP 705. A Digital to Analog Converter (DAC) 743 converts the signal and the resulting output is transmitted to the user through the speaker 745, all under control of a Main Control Unit (MCU) 703 which can be implemented as a Central Processing Unit (CPU) (not shown).

The MCU 703 receives various signals including input signals from the keyboard 747. The keyboard 747 and/or the MCU 703 in combination with other user input components (e.g., the microphone 711) comprise a user interface circuitry for managing user input. The MCU 703 runs a user interface software to facilitate user control of at least some functions of the mobile terminal 701 as described herein. The MCU 703 also delivers a display command and a switch command to the display 707 and to the speech output switching controller, respectively. Further, the MCU 703 exchanges information with the DSP 705 and can access an optionally incorporated SIM card 749 and a memory 751. In addition, the MCU 703 executes various control functions required of the terminal. The DSP 705 may, depending upon the implementation, perform any of a variety of conventional digital processing functions on the voice signals. Additionally, DSP 705 determines the background noise level of the local environment from the signals detected by microphone 711 and sets the gain of microphone 711 to a level selected to compensate for the natural tendency of the user of the mobile terminal 701.

The CODEC 713 includes the ADC 723 and DAC 743. The memory 751 stores various data including call incoming tone data and is capable of storing other data including music data received via, e.g., the global Internet. The software module could reside in RAM memory, flash memory, registers, or any other form of writable storage medium known in the art. The memory device 751 may be, but not limited to, a single memory, CD, DVD, ROM, RAM, EEPROM, optical storage, magnetic disk storage, flash memory storage, or any other non-volatile storage medium capable of storing digital data.

An optionally incorporated SIM card 749 carries, for instance, important information, such as the cellular phone number, the carrier supplying service, subscription details, and security information. The SIM card 749 serves primarily to identify the mobile terminal 701 on a radio network. The card 749 also contains a memory for storing a personal telephone number registry, text messages, and user specific mobile terminal settings.

In some embodiments, the mobile terminal 701 includes a digital camera comprising an array of optical detectors, such as charge coupled device (CCD) array 765. The output of the array is image data that is transferred to the MCU for further processing or storage in the memory 751 or both. In the illustrated embodiment, the light impinges on the optical array through a lens 763, such as a pin-hole lens or a material lens made of an optical grade glass or plastic material. In the illustrated embodiment, the mobile terminal 701 includes a light source 761, such as a LED to illuminate a subject for capture by the optical array, e.g., CCD 765. The light source is powered by the battery interface and power control module 720 and controlled by the MCU 703 based on instructions stored or loaded into the MCU 703.

In the foregoing specification, the invention has been described with reference to specific embodiments thereof. It will, however, be evident that various modifications and changes may be made thereto without departing from the broader spirit and scope of the invention. The specification and drawings are, accordingly, to be regarded in an illustrative rather than a restrictive sense. Throughout this specification and the claims, unless the context requires otherwise, the word "comprise" and its variations, such as "comprises" and "comprising," will be understood to imply the inclusion of a stated item, element or step or group of items, elements or steps but not the exclusion of any other item, element or step or group of items, elements or steps. Furthermore, the indefinite article "a" or "an" is meant to indicate one or more of the items, elements or steps modified by the article. As used herein, unless otherwise clear from the context, a value is "about" another value if it is within a factor of two (twice or half) of the other value. While example ranges are given, unless otherwise clear from the context, any contained ranges are also intended in various embodiments. Thus, a range from 0 to 10 includes the range 1 to 4 in some embodiments.

## Claims

1. A method comprising:
a. capturing, with a camera, one or more images of an item on a menu at an eating establishment;
b. performing step a for each item on the menu at the eating establishment;
c. performing step b for each menu at the eating establishment;
d. performing step c for each eating establishment of a plurality of eating establishments; and
e. storing, in a database, the images captured during steps a-d with data indicating the item, the menu and the eating establishment for each stored image.

2. The method of claim 1, wherein the database includes a data structure having a plurality of records for a respective plurality of eating establishments in the network, wherein each record includes a plurality of fields for holding the data indicating the item, the menu and the eating establishment for each stored image.

3. The method of claim 2, wherein each record includes a first field for holding data indicating the eating establishment, a second field for holding data indicating the menu, a third field for holding data indicating the item and a fourth field holding the stored image data.

4. The method of claim 3, wherein the third field holds data indicating a description of the item in a plurality of languages.

5. The method of claim 3, wherein the third field holds data indicating at least one of a name of the item, a price of the item, a food category of the item, a rating of the item and nutritional information of the item.

6. The method of claim 5, wherein the food category comprises one of vegan, vegetarian and non-vegetarian and wherein the nutritional information comprises ingredients, calories and information on fat, carbohydrates and protein.

7. The method of claim 3, wherein the first field is for holding a QR code indicating the eating establishment.

8. The method of claim 1, further comprising:
scanning, with a device, data indicating an eating establishment;
transmitting, from the device to the database, the scanned data indicating the eating establishment;
receiving, from the database to the device, the images of each item and each menu of the eating establishment based on the scanned data indicating the eating establishment; and
displaying, on a screen of the device, one or more images of received images on the device.

9. The method of claim 8, wherein the scanning comprises scanning, with a camera of the device, a QR code of the eating establishment.

10. A method for displaying images of menu items from one of a plurality of eating establishments, said method comprising:
scanning, with a device, data indicating one of the plurality of eating establishments;
transmitting, from the device to a database over a network, the scanned data;
receiving, from the database, one or more images of menu items at the eating establishment corresponding to the scanned data and data indicating the item, the menu and the eating establishment for each image;
displaying, in a first region of a screen of the device, the data indicating the eating establishment comprising a name of the eating establishment;
displaying, in a second region of the screen of the device, the data indicating the menu comprising a plurality of tabs indicating a respective plurality of menus of the eating establishment; and
displaying in a third region of the screen of the device, one or more images of menu items corresponding to one of the menus in the second region.

11. The method of claim 10, further comprising selecting one of the tabs in the second region to select one of the menus and wherein the displaying in the third region comprises displaying one or more images of menu items corresponding to the selected tab.

12. The method of claim 10, wherein upon selecting one of the images in the third region, said method includes:
displaying, in the first region, the selected image of the menu item;
displaying, in the second region, data indicating the item comprising at least one of a name of the item, a food category of the item and a rating of the item;
displaying, in the third region, data indicating the item comprising a written description of the item in one of a plurality of languages; and
displaying, in a fourth region, data indicating the item comprising nutritional information comprising at least one of calories and ingredients.

13. The method of claim 12, wherein the method further comprises displaying a plurality of active areas corresponding to the plurality of languages and selecting one of the active areas corresponding to a selected language and wherein the displaying in the third region comprises displaying the written description in the selected language.

14. A system comprising:
a camera at each of a plurality of eating establishments, wherein said camera is configured to capture one or more images of each item on each menu of the eating establishment;
a processor at each of the plurality of eating establishments, each processor communicatively coupled to the camera, said processor configured to receive the captured images from the camera; and
a database communicatively coupled with the processor of each of the plurality of eating establishments to receive the one or more images and data indicating the item, the menu and the eating establishment from each processor;
wherein said database includes a plurality of records for the respective plurality of eating establishments, wherein each record includes a plurality of fields for holding the data indicating the item, the menu and the eating establishment for each stored image.

15. The system of claim 14, further comprising:
a device configured to scan data indicating the eating establishment and further configured to transmit the scanned data to the database; and
a screen on the device configured to display one or more images received from the database of each item and each menu of the eating establishment based on the scanned data transmitted to the database.
